# EUROPÄISCHE PATENTSCHRIFT

(11) **EP 3 984 463 B1**
(45) Veröffentlichungstag und Bekanntmachung des Hinweises auf die Patenterteilung: **13.03.2024**
(21) Anmeldenummer: 21166392.7
(22) Anmeldetag: 31.03.2021
(51) Int. Cl.: A61B 6/03, A61B 6/00

(54) **GANTRY FÜR EIN COMPUTERTOMOGRAPHIEGERÄT UND VERFAHREN ZUM KÜHLEN EINES COMPUTERTOMOGRAPHIEGERÄTS**
GANTRY FOR A COMPUTER TOMOGRAPHY DEVICE AND METHOD FOR COOLING A COMPUTER TOMOGRAPHY DEVICE
PORTIQUE POUR UN APPAREIL DE TOMOGRAPHIE ASSISTÉE PAR ORDINATEUR ET PROCÉDÉ DE REFROIDISSEMENT D'UN APPAREIL DE TOMOGRAPHIE ASSISTÉE PAR ORDINATEUR

(43) Veröffentlichungstag der Anmeldung: 20.04.2022
(73) Patentinhaber: Siemens Healthineers AG, 91301 Forchheim (DE)
(72) Erfinder: Distler, Friedrich, 90766 Fürth (DE); Dietrich, Florian, 91301 Forchheim (DE); Hupfauf, Matthias, 92507 Nabburg (DE); Kühn, Ulrich, 91083 Baiersdorf (DE)
(74) Vertreter: Siemens Healthineers Patent Attorneys

(56) Entgegenhaltungen:
- DE-A1-102005 041 538
- DE-A1-102014 204 794
- DE-A1-102019 204 501

## Beschreibung

Die Erfindung betrifft eine Gantry für ein Computertomographiegerät und ein Verfahren zum Kühlen eines Computertomographiegeräts.

In einer Gantry eines Computertomographiegeräts (CT-Gerät) sollen zum einen sehr viele elektrische und elektronische Komponenten integriert sein. Zum anderen soll die Gantry des Computertomographiegeräts möglichst klein und kompakt sein. Dadurch kann es innerhalb der Gantry zu einer hohen Wärmeentwicklung kommen.

DE 10 2014 204 794 A1 offenbart ein CT-System zur Erzeugung tomographischer Aufnahmen eines Untersuchungsobjektes, wobei das CT-System eine Gantry mit einem rotierbaren Träger zur Aufnahme von Komponenten des CT-Systems und ein Kühlsystem zum Kühlen der auf der Gantry befestigten Komponenten mit mindestens einem luftführenden Kanal aufweist.

Die Erfindung hat die Aufgabe, eine Alternative zu herkömmlichen Kühlungskonzepten für eine Gantry eines Computertomographiegeräts bereitzustellen.

Die Erfindung betrifft eine Gantry für ein Computertomographiegerät,
- wobei die Gantry einen Tragrahmen, ein Drehlager und einen Drehrahmen aufweist,
- wobei der Drehrahmen mittels des Drehlagers um eine Drehachse relativ zu dem Tragrahmen drehbar gelagert ist und einen ersten Satz von Drehrahmen-Komponenten aufweist,
- wobei der Tragrahmen einen Lüfter zum Erzeugen eines Luftstroms, einen Druckkanal zum Führen des Luftstroms entlang wenigstens eines Strömungspfads und eine erste Tragrahmen-

Komponente aufweist, wobei der wenigstens eine Strömungspfad um die Drehachse herum gekrümmt ist,
- wobei der Druckkanal eine erste Druckkanalwand aufweist, die den Druckkanal in einer ersten Axialrichtung begrenzt, wobei die erste Axialrichtung zu der Drehachse im Wesentlichen parallel ist, insbesondere parallel ist, wobei die erste Druckkanalwand einen ersten Satz von drehrahmenseitigen Öffnungen aufweist, welcher dazu eingerichtet ist, von dem Luftstrom einen ersten Drehrahmen-Luftstrom abzuzweigen, wobei der erste Drehrahmen-Luftstrom zum Kühlen des ersten Satzes von Drehrahmen-Komponenten vorgesehen ist,
- wobei der Druckkanal eine zweite Druckkanalwand aufweist, die den Druckkanal in wenigstens einer Radialrichtung begrenzt, wobei die wenigstens eine Radialrichtung zu der Drehachse im Wesentlichen senkrecht ist, insbesondere senkrecht ist, wobei die zweite Druckkanalwand eine erste Öffnung aufweist, welche dazu eingerichtet ist, von dem Luftstrom einen ersten Tragrahmen-Luftstrom abzuzweigen, wobei der erste Tragrahmen-Luftstrom zum Kühlen der ersten Tragrahmen-Komponente vorgesehen ist.

Weiterhin kann vorgesehen sein, dass der erste Satz von drehrahmenseitigen Öffnungen wenigstens eine drehrahmenseitige Öffnung, die entlang des wenigstens einen Strömungspfads nach der ersten Öffnung angeordnet ist, insbesondere derart angeordnet ist, dass die erste Öffnung vor dieser wenigstens einen drehrahmenseitigen Öffnung und nach dem Lüfter angeordnet ist, enthält. Weiterhin kann vorgesehen sein, dass der erste Satz von drehrahmenseitigen Öffnung wenigstens eine drehrahmenseitige Öffnung, die entlang des wenigstens einen Strömungspfads vor der ersten Öffnung, insbesondere vor der ersten Öffnung und nach dem Lüfter, angeordnet ist, enthält.

Der Lüfter kann insbesondere ein Radiallüfter, beispielsweise ein vorwärts gekrümmter Radiallüfter oder ein rückwärts gekrümmter Radiallüfter, sein. Beispielsweise kann eine Drehachse des Radiallüfters, insbesondere eine Drehachse eines Radiallaufrads des Radiallüfters, parallel zu der Drehachse des Drehrahmens sein.

Eine Ausführungsform sieht vor, dass die erste Tragrahmen-Komponente ein elektronisches Gerät ist, insbesondere ein mikroelektronisches Gerät ist.

Weiterhin kann vorgesehen sein, dass die erste Tragrahmen-Komponente eine Datenverarbeitungseinheit zur Steuerung der Gantry und/oder eine Bildverarbeitungseinheit zur Verarbeitung von mittels des Computertomographiegeräts aufgenommenen Bilddaten ist.

Weiterhin kann vorgesehen sein, dass die erste Tragrahmen-Komponente ein leistungselektronisches Gerät und/oder ein Transformator zur Versorgung der Gantry mit einer elektrischen Leistung ist. Die erste Tragrahmen-Komponente kann beispielsweise ein Filtern zum Filtern elektrischer Störungen und/oder eine Drossel zur Versorgung der Gantry mit einer elektrischen Leistung sein.

Weiterhin kann vorgesehen sein, dass die erste Tragrahmen-Komponente ein Frequenzumrichter, beispielsweise für einen Drehantrieb zum Antreiben einer Drehbewegung des Drehrahmens um die Drehachse relativ zu dem Tragrahmen, und/oder ein Bremswiderstand, beispielsweise zum Bremsen der Drehbewegung des Drehrahmens um die Drehachse relativ zu dem Tragrahmen, ist.

Weiterhin kann vorgesehen sein, dass der Drehrahmen eine erste Drehrahmenwand aufweist, die in der ersten Axialrichtung auf die erste Druckkanalwand folgend angeordnet ist und/oder die den Drehrahmen in einer zweiten Axialrichtung begrenzt, wobei die zweite Axialrichtung zu der ersten Axialrichtung entgegengesetzt gerichtet ist. Die erste Drehrahmenwand kann sich insbesondere flächig senkrecht zu der Drehachse erstrecken.

Insbesondere kann vorgesehen sein, dass die erste Drehrahmenwand einen ersten Satz von druckkanalseitigen Öffnungen aufweist, welcher dazu eingerichtet ist, den ersten Drehrahmen-Luftstrom aufzunehmen, insbesondere nach einem Verlassen des Druckkanals durch den ersten Satz von drehrahmenseitigen Öffnungen und/oder während einer Drehbewegung des Drehrahmens um die Drehachse relativ zu dem Tragrahmen aufzunehmen.

Weiterhin kann vorgesehen sein, dass der Drehrahmen einen zweiten Satz von Drehrahmen-Komponenten aufweist, wobei der Druckkanal eine dritte Druckkanalwand aufweist, die den Druckkanal in einer zweiten Axialrichtung begrenzt, wobei die zweite Axialrichtung zu der Drehachse im Wesentlichen parallel ist und zu der ersten Axialrichtung entgegengesetzt gerichtet ist, wobei die dritte Druckkanalwand einen zweiten Satz von drehrahmenseitigen Öffnungen aufweist, welcher dazu eingerichtet ist, von dem Luftstrom einen zweiten Drehrahmen-Luftstrom abzuzweigen, wobei der zweite Drehrahmen-Luftstrom zum Kühlen des zweiten Satzes von Drehrahmen-Komponenten vorgesehen ist.

Weiterhin kann vorgesehen sein, dass der Drehrahmen eine zweite Drehrahmenwand aufweist, die in der zweiten Axialrichtung auf die dritte Druckkanalwand folgend angeordnet ist und/oder die den Drehrahmen in der ersten Axialrichtung begrenzt, wobei die zweite Drehrahmenwand einen zweiten Satz druckkanalseitiger Öffnungen aufweist, welcher dazu eingerichtet ist, den zweiten Drehrahmen-Luftstrom aufzunehmen, insbesondere nach einem Verlassen des Druckkanals durch den zweiten Satz von drehrahmenseitigen Öffnungen und/oder während einer Drehbewegung des Drehrahmens um die Drehachse relativ zu dem Tragrahmen aufzunehmen.

Der erste Satz von Drehrahmen-Komponenten kann beispielsweise wenigstens eine Röntgenstrahlungsquelle und/oder wenigstens einen Röntgendetektor aufweisen. Der zweite Satz von Drehrahmen-Komponenten kann beispielsweise wenigstens eine Röntgenstrahlungsquelle und/oder wenigstens einen Röntgendetektor aufweisen.

Insbesondere kann vorgesehen sein, dass der erste Satz von Drehrahmen-Komponenten ein Paar von Röntgenstrahlungsquellen und ein Paar von Röntgendetektoren eines Dual-Source-Computertomographiegeräts aufweist oder dass der zweite Satz von Drehrahmen-Komponenten ein Paar von Röntgenstrahlungsquellen und ein Paar von Röntgendetektoren eines Dual-Source-Computertomographiegeräts aufweist.

Eine Ausführungsform sieht vor, dass der Tragrahmen eine zweite Tragrahmen-Komponente aufweist, wobei die zweite Druckkanalwand eine zweite Öffnung aufweist, welche dazu eingerichtet ist, von dem Luftstrom einen zweiten Tragrahmen-Luftstrom abzuzweigen, wobei der zweite Tragrahmen-Luftstrom zum Kühlen der zweiten Tragrahmen-Komponente vorgesehen ist, wobei die erste Öffnung und die zweite Öffnung entlang des wenigstens einen Strömungspfads aufeinanderfolgend angeordnet sind.

Eine Ausführungsform sieht vor, dass die zweite Tragrahmen-Komponente ein elektronisches Gerät ist, insbesondere ein mikroelektronisches Gerät ist.

Weiterhin kann vorgesehen sein, dass die zweite Tragrahmen-Komponente eine Datenverarbeitungseinheit zur Steuerung der Gantry und/oder eine Bildverarbeitungseinheit zur Verarbeitung von mittels des Computertomographiegeräts aufgenommenen Bilddaten ist.

Weiterhin kann vorgesehen sein, dass die zweite Tragrahmen-Komponente ein leistungselektronisches Gerät und/oder ein Transformator zur Versorgung der Gantry mit einer elektrischen Leistung ist. Die zweite Tragrahmen-Komponente kann beispielsweise ein Filtern zum Filtern elektrischer Störungen und/oder eine Drossel zur Versorgung der Gantry mit einer elektrischen Leistung sein.

Weiterhin kann vorgesehen sein, dass die zweite Tragrahmen-Komponente ein Frequenzumrichter, beispielsweise für einen Drehantrieb zum Antreiben einer Drehbewegung des Drehrahmens um die Drehachse relativ zu dem Tragrahmen, und/oder ein Bremswiderstand, beispielsweise zum Bremsen der Drehbewegung des Drehrahmens um die Drehachse relativ zu dem Tragrahmen, ist.

Insbesondere kann vorgesehen sein, dass die erste Tragrahmen-Komponente ein mikroelektronisches Gerät und/oder eine Datenverarbeitungseinheit zur Steuerung der Gantry ist und dass die zweite Tragrahmen-Komponente ein leistungselektronisches Gerät und/oder ein Transformator zur Versorgung der Gantry mit einer elektrischen Leistung ist.

Eine Ausführungsform sieht vor, dass der erste Satz von drehrahmenseitigen Öffnungen wenigstens eine drehrahmenseitige Öffnung, die entlang des wenigstens einen Strömungspfads zwischen der ersten Öffnung und der zweiten Öffnung angeordnet ist, insbesondere vor der ersten Öffnung und nach der zweiten Öffnung angeordnet ist, enthält.

Eine Ausführungsform sieht vor, dass der Tragrahmen einen Kipprahmen, ein Kipplager und einen Stützrahmen aufweist, wobei der Kipprahmen mittels des Kipplagers um eine Kippachse relativ zu dem Stützrahmen kippbar gelagert ist, wobei der Kipprahmen den Lüfter und den Druckkanal aufweist, wobei der Drehrahmen mittels des Drehlagers um eine Drehachse relativ zu dem Kipprahmen drehbar gelagert ist, wobei der Stützrahmen die erste Tragrahmen-Komponente aufweist, wobei die erste Tragrahmen-Komponente mit der ersten Öffnung mittels einer Leitung zum Übertragen des ersten Tragrahmen-Luftstroms verbunden ist, wobei die Leitung einen flexiblen Abschnitt und/oder eine Drehdurchführung aufweist, um einer Kippbewegung des Kipprahmens relativ zu dem Stützrahmen folgen zu können.

Weiterhin kann vorgesehen sein, dass die Gantry eine Verkleidung zur Abgrenzung eines Innenbereichs der Gantry gegen eine Umgebung der Gantry aufweist, wobei der Innenbereich der Gantry einen Zwischenraum aufweist, wobei sich der Zwischenraum zwischen dem Tragrahmen und dem Drehrahmen einerseits und der Verkleidung andererseits erstreckt.

Insbesondere kann vorgesehen sein, dass der der Zwischenraum dazu eingerichtet ist, einen von dem ersten Satz von Drehrahmen-Komponenten kommenden Warmluftstrom und einen von der ersten Tragrahmen-Komponente kommenden Warmluftstrom aufzunehmen und zu dem Lüfter zu führen.

Insbesondere kann vorgesehen sein, dass der Zwischenraum ferner dazu eingerichtet ist, einen von dem zweiten Satz von Drehrahmen-Komponenten kommenden Warmluftstrom und/oder einen von der zweiten Tragrahmen-Komponente kommenden Warmluftstrom aufzunehmen und zu dem Lüfter zu führen.

Die Erfindung betrifft ferner ein Verfahren zum Kühlen eines Computertomographiegeräts,
- wobei das Computertomographiegerät eine Gantry mit einem Tragrahmen, einem Drehlager und einem Drehrahmen aufweist,
- wobei der Drehrahmen mittels des Drehlagers um eine Drehachse relativ zu dem Tragrahmen drehbar gelagert ist und einen ersten Satz von Drehrahmen-Komponenten aufweist,
- wobei der Tragrahmen einen Lüfter, einen Druckkanal und eine erste Tragrahmen-Komponente aufweist, wobei der Druckkanal eine erste Druckkanalwand aufweist, die den Druckkanal in einer ersten Axialrichtung begrenzt, wobei die erste Axialrichtung zur der Drehachse im Wesentlichen parallel ist, insbesondere parallel ist, wobei die erste Druckkanalwand einen ersten Satz von drehrahmenseitigen Öffnungen aufweist,
- wobei der Druckkanal eine zweite Druckkanalwand aufweist, die den Druckkanal in wenigstens einer Radialrichtung begrenzt, wobei die wenigstens eine Radialrichtung zu der Drehachse im Wesentlichen senkrecht ist, insbesondere senkrecht ist, wobei die zweite Druckkanalwand eine erste Öffnung aufweist,
- wobei ein Luftstrom mittels des Lüfters erzeugt wird,
- wobei der Luftstrom mittels des Druckkanals entlang wenigstens eines Strömungspfads geführt wird, wobei der wenigstens eine Strömungspfad um die Drehachse herum gekrümmt ist,
- wobei ein erster Drehrahmen-Luftstrom mittels des ersten Satzes von drehrahmenseitigen Öffnungen von dem Luftstrom abgezweigt wird,
- wobei der erste Satz von Drehrahmen-Komponenten mittels des ersten Drehrahmen-Luftstroms gekühlt wird,
- wobei ein erster Tragrahmen-Luftstrom mittels der ersten Öffnung von dem Luftstrom abgezweigt wird,
- wobei die erste Tragrahmen-Komponente mittels des ersten Tragrahmen-Luftstroms gekühlt wird.

Eine Ausführungsform sieht vor, dass die erste Tragrahmen-Komponente ein elektronisches Gerät ist, insbesondere ein mikroelektronisches Gerät ist.

Weiterhin kann vorgesehen sein, dass der Drehrahmen eine erste Drehrahmenwand aufweist, die in der ersten Axialrichtung auf die erste Druckkanalwand folgend angeordnet ist und/oder die den Drehrahmen in einer zweiten Axialrichtung begrenzt, wobei die zweite Axialrichtung zu der ersten Axialrichtung entgegengesetzt gerichtet ist.

Weiterhin kann vorgesehen sein, dass die erste Drehrahmenwand einen ersten Satz von druckkanalseitigen Öffnungen aufweist, wobei der erste Drehrahmen-Luftstrom mittels des ersten Satzes von druckkanalseitigen Öffnungen aufgenommen wird, insbesondere nach einem Verlassen des Druckkanals durch den ersten Satz von drehrahmenseitigen Öffnungen und/oder während einer Drehbewegung des Drehrahmens um die Drehachse relativ zu dem Tragrahmen aufgenommen wird.

Eine Ausführungsform sieht vor, dass der Drehrahmen einen zweiten Satz von Drehrahmen-Komponenten aufweist, wobei der Druckkanal eine dritte Druckkanalwand aufweist, die den Druckkanal in einer zweiten Axialrichtung begrenzt, wobei die zweite Axialrichtung zu der Drehachse im Wesentlichen parallel ist und zu der ersten Axialrichtung entgegengesetzt gerichtet ist, wobei die dritte Druckkanalwand einen zweiten Satz von drehrahmenseitigen Öffnungen aufweist,
- wobei ein zweiter Drehrahmen-Luftstrom mittels des zweiten Satzes von drehrahmenseitigen Öffnungen von dem Luftstrom abgezweigt wird,
- wobei der Drehrahmen eine zweite Drehrahmenwand aufweist, die in der zweiten Axialrichtung auf die dritte Druckkanalwand folgend angeordnet ist, wobei die zweite Drehrahmenwand einen zweiten Satz druckkanalseitiger Öffnungen aufweist,
- wobei der zweite Drehrahmen-Luftstrom mittels des zweiten Satzes druckkanalseitiger Öffnungen aufgenommen wird,
- wobei der zweite Satz von Drehrahmen-Komponenten mittels des zweiten Drehrahmen-Luftstroms gekühlt wird.

Eine Ausführungsform sieht vor, dass der Tragrahmen eine zweite Tragrahmen-Komponente aufweist, wobei die zweite Druckkanalwand eine zweite Öffnung aufweist,
- wobei ein zweiter Tragrahmen-Luftstrom mittels der zweiten Öffnung von dem Luftstrom abgezweigt wird, wobei die erste Öffnung und die zweite Öffnung entlang des wenigstens einen Strömungspfades aufeinanderfolgend angeordnet sind,
- wobei die zweite Tragrahmen-Komponente mittels des zweiten Tragrahmen-Luftstroms gekühlt wird.

Eine Ausführungsform sieht vor, dass der Tragrahmen einen Kipprahmen, ein Kipplager und einen Stützrahmen aufweist, wobei der Kipprahmen mittels des Kipplagers um eine Kippachse relativ zu dem Stützrahmen kippbar gelagert ist,
- wobei der Kipprahmen den Lüfter und den Druckkanal aufweist, wobei der Drehrahmen mittels des Drehlagers um eine Drehachse relativ zu dem Kipprahmen drehbar gelagert ist,
- wobei der Stützrahmen die erste Tragrahmen-Komponente aufweist,
- wobei die erste Tragrahmen-Komponente mit der ersten Öffnung mittels einer Leitung zum Übertragen des ersten Tragrahmen-Luftstroms verbunden ist, wobei die Leitung einen flexiblen Abschnitt und/oder eine Drehdurchführung aufweist,
- wobei eine Kippbewegung des Kipprahmens relativ zu dem Stützrahmen ausgeführt wird,
- wobei die Leitung mittels des flexiblen Abschnitts und/oder der Drehdurchführung der Kippbewegung des Kipprahmens relativ zu dem Stützrahmen folgt.

Die Kippbewegung des Kipprahmens relativ zu dem Stützrahmen kann insbesondere ausgeführt werden, indem die Kippbewegung des Kipprahmens relativ zu dem Stützrahmen mittels eines Kippantriebs angetrieben wird.

Eine Ausführungsform sieht vor, dass die Gantry eine Verkleidung zur Abgrenzung eines Innenbereichs der Gantry gegen eine Umgebung der Gantry aufweist,
- wobei der Innenbereich der Gantry einen Zwischenraum aufweist, wobei sich der Zwischenraum zwischen dem Tragrahmen und dem Drehrahmen einerseits und der Verkleidung andererseits erstreckt,
- wobei mittels des Zwischenraums ein von dem ersten Satz von Drehrahmen-Komponenten kommender Warmluftstrom und ein von der ersten Tragrahmen-Komponente kommender Warmluftstrom aufgenommen und zu dem Lüfter geführt werden.

Ferner kann mittels des Zwischenraums ein von dem zweiten Satz von Drehrahmen-Komponenten kommender Warmluftstrom und/oder ein von der zweiten Tragrahmen-Komponente kommender Warmluftstrom aufgenommen und zu dem Lüfter geführt werden.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.

Die Fig. 1 zeigt ein Computertomographiegerät mit einer Gantry und der Verkleidung C.

Die Fig. 2 zeigt die Gantry für das Computertomographiegerät.

Die Fig. 3 zeigt die Gantry für das Computertomographiegerät in einer weiteren Ansicht.

Die Fig. 4 zeigt die Gantry für das Computertomographiegerät, wobei das Computertomographiegerät ein Dual-Source-Computertomographiegerät ist.

Die Fig. 5 zeigt die Gantry für das Computertomographiegerät mit einem Kipprahmen.

Die Fig. 6 zeigt die Gantry für das Computertomographiegerät in einer weiteren Ansicht.

Die Fig. 7 zeigt ein Ablaufdiagramm für ein Verfahren zum Kühlen eines Computertomographiegeräts.

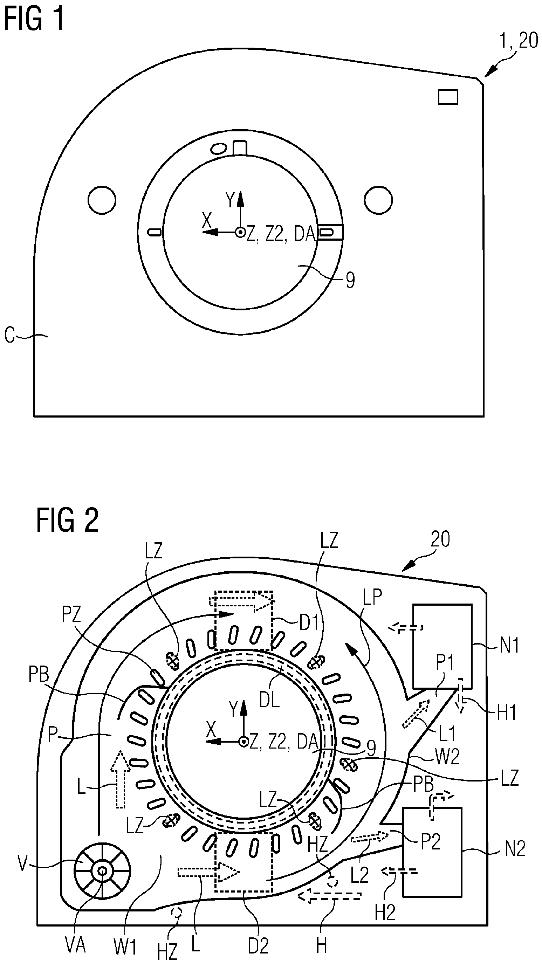

Die Fig. 1 zeigt ein Computertomographiegerät 1 mit einer Gantry 20 und der Verkleidung C. Das Computertomographiegerät 1 weist eine Gantry 20 mit einer tunnelförmigen Öffnung 9 auf. Insbesondere kann in die tunnelförmige Öffnung 9 ein Patient eingeführt werden, um mittels des Computertomographiegeräts 1 untersucht zu werden.

Die Fig. 2 zeigt die Gantry 20 für das Computertomographiegerät 1, wobei die Gantry 20 einen Tragrahmen TR, ein Drehlager DL und einen Drehrahmen DR aufweist, wobei der Drehrahmen DR mittels des Drehlagers DL um eine Drehachse DA relativ zu dem Tragrahmen TR drehbar gelagert ist und einen ersten Satz von Drehrahmen-Komponenten D1, D2 aufweist.

Der Tragrahmen TR weist einen Lüfter V zum Erzeugen eines Luftstroms L, einen Druckkanal P zum Führen des Luftstroms L entlang des Strömungspfads LP und eine erste Tragrahmen-Komponente N1 auf. Der Strömungspfad LP ist um die Drehachse DA herum gekrümmt.

Der Druckkanal P weist eine erste Druckkanalwand W1 auf, die den Druckkanal P in einer ersten Axialrichtung Z begrenzt, wobei die erste Axialrichtung Z zu der Drehachse DA parallel ist, wobei die erste Druckkanalwand W1 einen ersten Satz von drehrahmenseitigen Öffnungen PZ aufweist, welcher dazu eingerichtet ist, von dem Luftstrom L einen ersten Drehrahmen-Luftstrom LZ abzuzweigen, wobei der erste Drehrahmen-Luftstrom LZ zum Kühlen des ersten Satzes von Drehrahmen-Komponenten D1, D2 vorgesehen ist.

Der Druckkanal P weist eine zweite Druckkanalwand W2 aufweist, die den Druckkanal P in wenigstens einer Radialrichtung X, Y begrenzt, wobei die wenigstens eine Radialrichtung X, Y zu der Drehachse DA senkrecht ist, wobei die zweite Druckkanalwand W2 eine erste Öffnung P1 aufweist, welche dazu eingerichtet ist, von dem Luftstrom L einen ersten Tragrahmen-Luftstrom L1 abzuzweigen, wobei der erste Tragrahmen-Luftstrom L1 zum Kühlen der ersten Tragrahmen-Komponente N1 vorgesehen ist.

Die Drehachse DA ist eine Gerade. Die erste Druckkanalwand W1 erstreckt sich flächig senkrecht zu der Drehachse DA. Die zweite Druckkanalwand W2 erstreckt sich flächig senkrecht zu der ersten Druckkanalwand W1. Der erste Satz von drehrahmenseitigen Öffnungen PZ enthält mehrere drehrahmenseitige Öffnungen, die entlang des Strömungspfades aufeinanderfolgend angeordnet sind. Der erste Drehrahmen-Luftstrom LZ ist zusammengesetzt aus einer Mehrzahl von Teil-Luftströmen, wobei jeder Teil-Luftstrom der Mehrzahl von Teil-Luftströmen durch eine entsprechende drehrahmenseitige Öffnung des ersten Satzes von drehrahmenseitigen Öffnungen strömt.

Der erste Satz von drehrahmenseitigen Öffnungen PZ enthält wenigstens eine drehrahmenseitige Öffnung, die entlang des Strömungspfads LP nach der ersten Öffnung P1 angeordnet ist, insbesondere derart angeordnet ist, dass die erste Öffnung P1 vor dieser wenigstens einen drehrahmenseitigen Öffnung und nach dem Lüfter V angeordnet ist. Der erste Satz von drehrahmenseitigen Öffnung PZ enthält wenigstens eine drehrahmenseitige Öffnung, die entlang des Strömungspfads LP vor der ersten Öffnung P1 und nach dem Lüfter angeordnet ist.

Der Tragrahmen TR weist eine zweite Tragrahmen-Komponente N2 auf, wobei die zweite Druckkanalwand W2 eine zweite Öffnung P2 aufweist, welche dazu eingerichtet ist, von dem Luftstrom L einen zweiten Tragrahmen-Luftstrom L2 abzuzweigen, wobei der zweite Tragrahmen-Luftstrom L2 zum Kühlen der zweiten Tragrahmen-Komponente N2 vorgesehen ist, wobei die erste Öffnung P1 und die zweite Öffnung P2 entlang des Strömungspfads LP aufeinanderfolgend angeordnet sind.

Der erste Satz von drehrahmenseitigen Öffnungen PZ enthält wenigstens eine drehrahmenseitige Öffnung, die entlang des Strömungspfads LP zwischen der ersten Öffnung P1 und der zweiten Öffnung P2 angeordnet ist, insbesondere vor der ersten Öffnung P1 und nach der zweiten Öffnung P2 angeordnet ist.

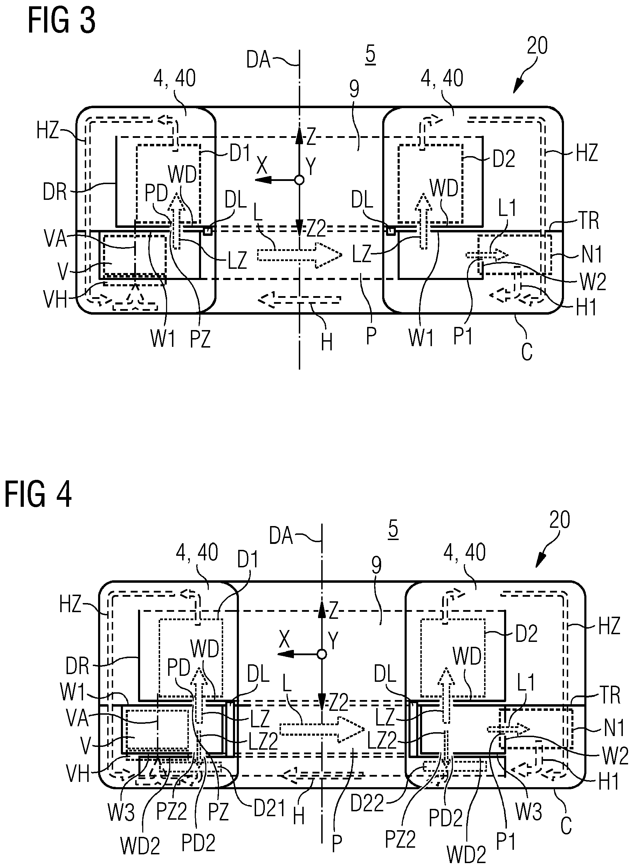

Die Fig. 3 zeigt die Gantry 20 für das Computertomographiegerät 1 in einer weiteren Ansicht. Der Drehrahmen DR weist eine erste Drehrahmenwand WD auf, die in der ersten Axialrichtung Z auf die erste Druckkanalwand W1 folgend angeordnet ist und die den Drehrahmen DR in der zweiten Axialrichtung Z2 begrenzt. Die zweite Axialrichtung Z2 ist zu der ersten Axialrichtung Z entgegengesetzt gerichtet. Die erste Drehrahmenwand WD erstreckt sich flächig senkrecht zu der Drehachse DA.

Die erste Drehrahmenwand WD weist einen ersten Satz von druckkanalseitigen Öffnungen PD auf, welcher dazu eingerichtet ist, den ersten Drehrahmen-Luftstrom LZ aufzunehmen, insbesondere nach einem Verlassen des Druckkanals P durch den ersten Satz von drehrahmenseitigen Öffnungen PZ und während einer Drehbewegung des Drehrahmens DR um die Drehachse DA relativ zu dem Tragrahmen TR aufzunehmen.

Die Fig. 4 zeigt die Gantry 20 für das Computertomographiegerät 1, wobei das Computertomographiegerät ein Dual-Source-Computertomographiegerät ist.

Der Drehrahmen DR weist einen zweiten Satz von Drehrahmen-Komponenten D21, D22 auf, wobei der Druckkanal P eine dritte Druckkanalwand W3 aufweist, die den Druckkanal P in einer zweiten Axialrichtung Z2 begrenzt, wobei die zweite Axialrichtung Z2 zu der Drehachse DA im Wesentlichen parallel ist und zu der ersten Axialrichtung Z entgegengesetzt gerichtet ist, wobei die dritte Druckkanalwand W3 einen zweiten Satz von drehrahmenseitigen Öffnungen PZ2 aufweist, welcher dazu eingerichtet ist, von dem Luftstrom L einen zweiten Drehrahmen-Luftstrom LZ2 abzuzweigen, wobei der zweite Drehrahmen-Luftstrom LZ2 zum Kühlen des zweiten Satzes von Drehrahmen-Komponenten D21, D22 vorgesehen ist.

Der Drehrahmen DR weist eine zweite Drehrahmenwand WD2 auf, die in der zweiten Axialrichtung Z2 auf die dritte Druckkanalwand W3 folgend angeordnet ist und die den Drehrahmen DR in der ersten Axialrichtung Z begrenzt, wobei die zweite Drehrahmenwand WD2 einen zweiten Satz druckkanalseitiger Öffnungen PD2 aufweist, welcher dazu eingerichtet ist, den zweiten Drehrahmen-Luftstrom LZ2 aufzunehmen, insbesondere nach einem Verlassen des Druckkanals P durch den zweiten Satz von drehrahmenseitigen Öffnungen PZ2 und während einer Drehbewegung des Drehrahmens DR um die Drehachse DA relativ zu dem Tragrahmen TR aufzunehmen. Die zweite Drehrahmenwand WD2 erstreckt sich flächig senkrecht zu der Drehachse DA.

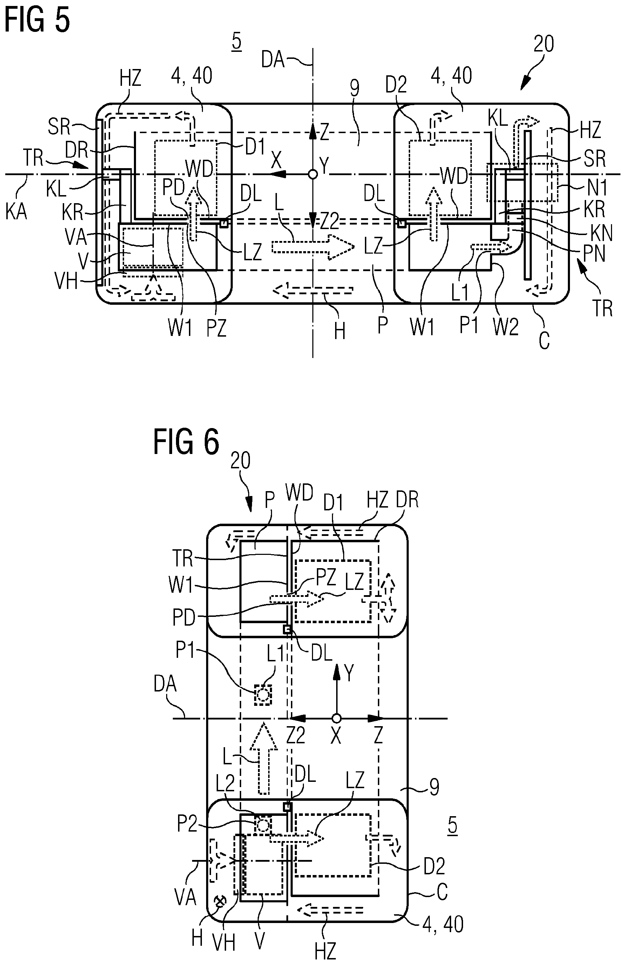

Die Fig. 5 zeigt die Gantry 20 für das Computertomographiegerät 1 mit einem Kipprahmen KR.

Der Tragrahmen TR weist den Kipprahmen KR, ein Kipplager KL und einen Stützrahmen SR auf, wobei der Kipprahmen KR mittels des Kipplagers KL um eine Kippachse KA relativ zu dem Stützrahmen SR kippbar gelagert ist,
- wobei der Kipprahmen den Lüfter V und den Druckkanal P aufweist, wobei der Drehrahmen DR mittels des Drehlagers DL um eine Drehachse DA relativ zu dem Kipprahmen KR drehbar gelagert ist,
- wobei der Stützrahmen SR die erste Tragrahmen-Komponente N1 aufweist,
- wobei die erste Tragrahmen-Komponente N1 mit der ersten Öffnung P1 mittels einer Leitung PN zum Übertragen des ersten Tragrahmen-Luftstroms L1 verbunden ist, wobei die Leitung PN einen flexiblen Abschnitt und/oder eine Drehdurchführung aufweist, um einer Kippbewegung des Kipprahmens KR relativ zu dem Stützrahmen SR folgen zu können.

Die Gantry 20 weist den Kippantrieb KN auf, der dazu eingerichtet ist, die Kippbewegung des Kipprahmens KR relativ zu dem Stützrahmen SR anzutreiben.

Die Fig. 6 zeigt die Gantry 20 für das Computertomographiegerät 1 in einer weiteren Ansicht. Die Gantry 20 weist eine Verkleidung C zur Abgrenzung eines Innenbereichs 4 der Gantry 20 gegen eine Umgebung 5 der Gantry auf, wobei der Innenbereich 4 der Gantry 20 einen Zwischenraum 40 aufweist, wobei sich der Zwischenraum 40 zwischen dem Tragrahmen TR und dem Drehrahmen DR einerseits und der Verkleidung C andererseits erstreckt.

Der Zwischenraum 40 ist dazu eingerichtet, einen von dem ersten Satz von Drehrahmen-Komponenten D1, D2 kommenden Warmluftstrom und einen von der ersten Tragrahmen-Komponente N1 kommenden Warmluftstrom H1 aufzunehmen und zu dem Lüfter V zu führen.

Der Zwischenraum 40 ist ferner dazu eingerichtet, einen von dem zweiten Satz von Drehrahmen-Komponenten D21, D22 kommenden Warmluftstrom und einen von der zweiten Tragrahmen-Komponente N2 kommenden Warmluftstrom H2 aufzunehmen und zu dem Lüfter V zu führen

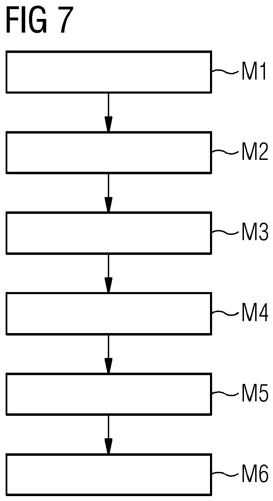

Die Fig. 7 zeigt ein Ablaufdiagramm für ein Verfahren zum Kühlen eines Computertomographiegeräts 1,
- wobei das Computertomographiegerät 1 eine Gantry 20 mit einem Tragrahmen TR, einem Drehlager DL und einem Drehrahmen DR aufweist,
- wobei der Drehrahmen DR mittels des Drehlagers DL um eine Drehachse DA relativ zu dem Tragrahmen TR drehbar gelagert ist und einen ersten Satz von Drehrahmen-Komponenten D1, D2 aufweist,
- wobei der Tragrahmen TR einen Lüfter V, einen Druckkanal P und eine erste Tragrahmen-Komponente N1 aufweist, wobei der Druckkanal P eine erste Druckkanalwand W1 aufweist, die den Druckkanal P in einer ersten Axialrichtung Z begrenzt, wobei die erste Axialrichtung Z zur der Drehachse DA parallel ist, wobei die erste Druckkanalwand W1 einen ersten Satz von drehrahmenseitigen Öffnungen PZ aufweist,
- wobei der Druckkanal P eine zweite Druckkanalwand W2 aufweist, die den Druckkanal P in wenigstens einer Radialrichtung X, Y begrenzt, wobei die wenigstens eine Radialrichtung X, Y zu der Drehachse DA senkrecht ist, wobei die zweite Druckkanalwand W2 eine erste Öffnung P1 aufweist,
- wobei ein Luftstrom L mittels des Lüfters V erzeugt M1 wird,
- wobei der Luftstrom L mittels des Druckkanals P entlang des Strömungspfads LP geführt M2 wird, wobei der Strömungspfad LP um die Drehachse DA herum gekrümmt ist,
- wobei ein erster Drehrahmen-Luftstrom LZ mittels des ersten Satzes von drehrahmenseitigen Öffnungen PZ von dem Luftstrom L abgezweigt M3 wird,
- wobei der erste Satz von Drehrahmen-Komponenten D1, D2 mittels des ersten Drehrahmen-Luftstroms LZ gekühlt M4 wird,
- wobei ein erster Tragrahmen-Luftstrom L1 mittels der ersten Öffnung P1 von dem Luftstrom L abgezweigt M5 wird,
- wobei die erste Tragrahmen-Komponente N1 mittels des ersten Tragrahmen-Luftstroms L1 gekühlt M6 wird.

Der Drehrahmen DR weist eine erste Drehrahmenwand WD auf, die in der ersten Axialrichtung Z auf die erste Druckkanalwand W1 folgend angeordnet ist und die den Drehrahmen DR in einer zweiten Axialrichtung Z2 begrenzt, wobei die zweite Axialrichtung Z2 zu der ersten Axialrichtung Z entgegengesetzt gerichtet ist.

Die erste Drehrahmenwand WD weist einen ersten Satz von druckkanalseitigen Öffnungen PD auf. Der erste Drehrahmen-Luftstrom LZ wird mittels des ersten Satzes von druckkanalseitigen Öffnungen PD aufgenommen, insbesondere nach einem Verlassen des Druckkanals P durch den ersten Satz von drehrahmenseitigen Öffnungen PZ und während einer Drehbewegung des Drehrahmens DR um die Drehachse DA relativ zu dem Tragrahmen TR aufgenommen.

Der Drehrahmen DR weist einen zweiten Satz von Drehrahmen-Komponenten D21, D22 auf, wobei der Druckkanal P eine dritte Druckkanalwand W3 aufweist, die den Druckkanal P in einer zweiten Axialrichtung Z2 begrenzt, wobei die zweite Axialrichtung Z2 zu der Drehachse DA im Wesentlichen parallel ist und zu der ersten Axialrichtung Z entgegengesetzt gerichtet ist, wobei die dritte Druckkanalwand W3 einen zweiten Satz von drehrahmenseitigen Öffnungen PZ2 aufweist. Ein zweiter Drehrahmen-Luftstrom LZ2 wird mittels des zweiten Satzes von drehrahmenseitigen Öffnungen PZ2 von dem Luftstrom L abgezweigt.

Der Drehrahmen DR weist eine zweite Drehrahmenwand WD2 auf, die in der zweiten Axialrichtung Z2 auf die dritte Druckkanalwand W3 folgend angeordnet ist, wobei die zweite Drehrahmenwand WD2 einen zweiten Satz druckkanalseitiger Öffnungen PD2 aufweist. Der zweite Drehrahmen-Luftstrom LZ2 wird mittels des zweiten Satzes druckkanalseitiger Öffnungen PD2 aufgenommen. Der zweite Satz von Drehrahmen-Komponenten D21, D22 wird mittels des zweiten Drehrahmen-Luftstroms LZ2 gekühlt.

Der Tragrahmen TR weist eine zweite Tragrahmen-Komponente N2 auf, wobei die zweite Druckkanalwand W2 eine zweite Öffnung P2 aufweist. Der zweite Tragrahmen-Luftstrom L2 wird mittels der zweiten Öffnung P2 von dem Luftstrom L abgezweigt, wobei die erste Öffnung P1 und die zweite Öffnung P2 entlang des Strömungspfades LP aufeinanderfolgend angeordnet sind. Die zweite Tragrahmen-Komponente N2 wird mittels des zweiten Tragrahmen-Luftstroms L2 gekühlt.

Der Tragrahmen TR weist einen Kipprahmen KR, ein Kipplager KL und einen Stützrahmen SR auf, wobei der Kipprahmen KR mittels des Kipplagers KL um eine Kippachse KA relativ zu dem Stützrahmen SR kippbar gelagert ist, wobei der Kipprahmen KR den Lüfter V und den Druckkanal P aufweist, wobei der Drehrahmen DR mittels des Drehlagers DL um eine Drehachse DA relativ zu dem Kipprahmen KR drehbar gelagert ist, wobei der Stützrahmen SR die erste Tragrahmen-Komponente N1 aufweist, wobei die erste Tragrahmen-Komponente N1 mit der ersten Öffnung P1 mittels einer Leitung PN zum Übertragen des ersten Tragrahmen-Luftstroms L1 verbunden ist, wobei die Leitung PN einen flexiblen Abschnitt und/oder eine Drehdurchführung aufweist.

Die Kippbewegung des Kipprahmens KR wird relativ zu dem Stützrahmen SR ausgeführt. Die Leitung P folgt mittels des flexiblen Abschnitts und/oder der Drehdurchführung der Kippbewegung des Kipprahmens KR relativ zu dem Stützrahmen SR. Die Kippbewegung des Kipprahmens KR relativ zu dem Stützrahmen SR kann insbesondere ausgeführt werden, indem die Kippbewegung des Kipprahmens KR relativ zu dem Stützrahmen SR mittels des Kippantriebs KN angetrieben wird.

Die Gantry 20 weist eine Verkleidung C zur Abgrenzung eines Innenbereichs 4 der Gantry 20 gegen eine Umgebung 5 der Gantry auf, wobei der Innenbereich 4 der Gantry 20 einen Zwischenraum 40 aufweist, wobei sich der Zwischenraum 40 zwischen dem Tragrahmen TR und dem Drehrahmen DR einerseits und der Verkleidung C andererseits erstreckt.

Mittels des Zwischenraums 40 werden ein von dem ersten Satz von Drehrahmen-Komponenten D1, D2 kommender Warmluftstrom und ein von der ersten Tragrahmen-Komponente N1 kommender Warmluftstrom H1 aufgenommen und zu dem Lüfter V geführt. Ferner werden mittels des Zwischenraums 40 ein von dem zweiten Satz von Drehrahmen-Komponenten D21, D22 kommender Warmluftstrom und ein von der zweiten Tragrahmen-Komponente N2 kommender Warmluftstrom H2 aufgenommen und zu dem Lüfter V geführt. In dem Zwischenraum 40 werden insbesondere die Warmluftströme HZ und H gebildet.

Die Warmluft wird mittels des Lüfters V durch den wassergekühlten Wärmetauscher VH hindurch entlang der Lüfterachse VA angesaugt und dabei gekühlt. Der Lüfter V ist ein Radiallüfter, beispielsweise ein vorwärts gekrümmter Radiallüfter oder ein rückwärts gekrümmter Radiallüfter. Die Lüfterachse VA ist parallel zu der Drehachse DA. Die Lüfterachse VA ist die Drehachse des Radiallüfters, insbesondere eine Drehachse eines Radiallaufrads des Radiallüfters. Es sind jedoch auch Ausführungsformen, die nicht auf einem wassergekühlten Wärmetauscher und/oder nicht auf einem Radiallüfter basieren, denkbar.

Die Erfindung ermöglicht insbesondere, mit lediglich einem Lüfter sowohl Drehrahmen-Komponenten als auch Tragrahmen-Komponenten zu kühlen. Dadurch können Bauraum, Kosten, Fehleranfälligkeit und Wartungsaufwand reduziert werden.

## Patentansprüche

1. Gantry (20) für ein Computertomographiegerät (1),
- wobei die Gantry (20) einen Tragrahmen (TR), ein Drehlager (DL) und einen Drehrahmen (DR) aufweist,
- wobei der Drehrahmen (DR) mittels des Drehlagers (DL) um eine Drehachse (DA) relativ zu dem Tragrahmen (TR) drehbar gelagert ist und einen ersten Satz von Drehrahmen-Komponenten (D1, D2) aufweist,
- wobei der Tragrahmen (TR) einen Lüfter (V) zum Erzeugen eines Luftstroms (L), einen Druckkanal (P) zum Führen des Luftstroms (L) entlang wenigstens eines Strömungspfads (LP) und eine erste Tragrahmen-Komponente (N1) aufweist, wobei der wenigstens eine Strömungspfad (LP) um die Drehachse (DA) herum gekrümmt ist,
- wobei der Druckkanal (P) eine erste Druckkanalwand (W1) aufweist, die den Druckkanal (P) in einer ersten Axialrichtung (Z) begrenzt, wobei die erste Axialrichtung (Z) zu der Drehachse (DA) im Wesentlichen parallel ist, wobei die erste Druckkanalwand (W1) einen ersten Satz von drehrahmenseitigen Öffnungen (PZ) aufweist, welcher dazu eingerichtet ist, von dem Luftstrom (L) einen ersten Drehrahmen-Luftstrom (LZ) abzuzweigen, wobei der erste Drehrahmen-Luftstrom (LZ) zum Kühlen des ersten Satzes von Drehrahmen-Komponenten (D1, D2) vorgesehen ist,
- wobei der Druckkanal (P) eine zweite Druckkanalwand (W2) aufweist, die den Druckkanal (P) in wenigstens einer Radialrichtung (X, Y) begrenzt, wobei die wenigstens eine Radialrichtung (X, Y) zu der Drehachse (DA) im Wesentlichen senkrecht ist, **dadurch gekennzeichnet, dass** die zweite Druckkanalwand (W2) eine erste Öffnung (P1) aufweist, welche dazu eingerichtet ist, von dem Luftstrom (L) einen ersten Tragrahmen-Luftstrom (L1) abzuzweigen, wobei der erste Tragrahmen-Luftstrom (L1) zum Kühlen der ersten Tragrahmen-Komponente (N1) vorgesehen ist.

2. Gantry (20) nach Anspruch 1,
- wobei der erste Satz von drehrahmenseitigen Öffnungen (PZ) wenigstens eine drehrahmenseitige Öffnung, die entlang des wenigstens einen Strömungspfads (LP) nach der ersten Öffnung (P1) angeordnet ist, enthält,
- wobei der erste Satz von drehrahmenseitigen Öffnung (PZ) wenigstens eine drehrahmenseitige Öffnung, die entlang des wenigstens einen Strömungspfads (LP) vor der ersten Öffnung (P1) angeordnet ist, enthält.

3. Gantry (20) nach Anspruch 1 oder 2,
- wobei die erste Tragrahmen-Komponente (N1) ein elektronisches Gerät ist.

4. Gantry (20) nach einem der Ansprüche 1 bis 3,
- wobei der Drehrahmen (DR) eine erste Drehrahmenwand (WD) aufweist, die in der ersten Axialrichtung (Z) auf die erste Druckkanalwand (W1) folgend angeordnet ist,
- wobei die erste Drehrahmenwand (WD) einen ersten Satz von druckkanalseitigen Öffnungen (PD) aufweist, welcher dazu eingerichtet ist, den ersten Drehrahmen-Luftstrom (LZ) aufzunehmen.

5. Gantry (20) nach einem der Ansprüche 1 bis 4,
- wobei der Drehrahmen (DR) einen zweiten Satz von Drehrahmen-Komponenten (D21, D22) aufweist,
- wobei der Druckkanal (P) eine dritte Druckkanalwand (W3) aufweist, die den Druckkanal (P) in einer zweiten Axialrichtung (Z2) begrenzt, wobei die zweite Axialrichtung (Z2) zu der Drehachse (DA) im Wesentlichen parallel ist und zu der ersten Axialrichtung (Z) entgegengesetzt gerichtet ist, wobei die dritte Druckkanalwand (W3) einen zweiten Satz von drehrahmenseitigen Öffnungen (PZ2) aufweist, welcher dazu eingerichtet ist, von dem Luftstrom (L) einen zweiten Drehrahmen-Luftstrom (LZ2) abzuzweigen, wobei der zweite Drehrahmen-Luftstrom (LZ2) zum Kühlen des zweiten Satzes von Drehrahmen-Komponenten (D21, D22) vorgesehen ist,
- wobei der Drehrahmen (DR) eine zweite Drehrahmenwand (WD2) aufweist, die in der zweiten Axialrichtung (Z2) auf die dritte Druckkanalwand (W3) folgend angeordnet ist,
- wobei die zweite Drehrahmenwand (WD2) einen zweiten Satz druckkanalseitiger Öffnungen (PD2) aufweist, welcher dazu eingerichtet ist, den zweiten Drehrahmen-Luftstrom (LZ2) aufzunehmen.

6. Gantry (20) nach einem der Ansprüche 1 bis 5,
- wobei der Tragrahmen (TR) eine zweite Tragrahmen-Komponente (N2) aufweist,
- wobei die zweite Druckkanalwand (W2) eine zweite Öffnung (P2) aufweist, welche dazu eingerichtet ist, von dem Luftstrom (L) einen zweiten Tragrahmen-Luftstrom (L2) abzuzweigen, wobei der zweite Tragrahmen-Luftstrom (L2) zum Kühlen der zweiten Tragrahmen-Komponente (N2) vorgesehen ist,
- wobei die erste Öffnung (P1) und die zweite Öffnung (P2) entlang des wenigstens einen Strömungspfads (LP) aufeinanderfolgend angeordnet sind.

7. Gantry (20) nach Anspruch 6,
- wobei der erste Satz von drehrahmenseitigen Öffnungen (PZ) wenigstens eine drehrahmenseitige Öffnung, die entlang des wenigstens einen Strömungspfads (LP) zwischen der ersten Öffnung (P1) und der zweiten Öffnung (P2) angeordnet ist, enthält.

8. Gantry (20) nach einem der Ansprüche 1 bis 7,
- wobei der Tragrahmen (TR) einen Kipprahmen (KR), ein Kipplager (KL) und einen Stützrahmen (SR) aufweist, wobei der Kipprahmen (KR) mittels des Kipplagers (KL) um eine Kippachse (KA) relativ zu dem Stützrahmen (SR) kippbar gelagert ist,
- wobei der Kipprahmen den Lüfter (V) und den Druckkanal (P) aufweist, wobei der Drehrahmen (DR) mittels des Drehlagers (DL) um eine Drehachse (DA) relativ zu dem Kipprahmen (KR) drehbar gelagert ist,
- wobei der Stützrahmen (SR) die erste Tragrahmen-Komponente (N1) aufweist,
- wobei die erste Tragrahmen-Komponente (N1) mit der ersten Öffnung (P1) mittels einer Leitung (PN) zum Übertragen des ersten Tragrahmen-Luftstroms (L1) verbunden ist, wobei die Leitung (PN) einen flexiblen Abschnitt und/oder eine Drehdurchführung aufweist, um einer Kippbewegung des Kipprahmens (KR) relativ zu dem Stützrahmen (SR) folgen zu können.

9. Verfahren zum Kühlen eines Computertomographiegeräts (1),
- wobei das Computertomographiegerät (1) eine Gantry (20) mit einem Tragrahmen (TR), einem Drehlager (DL) und einem Drehrahmen (DR) aufweist,
- wobei der Drehrahmen (DR) mittels des Drehlagers (DL) um eine Drehachse (DA) relativ zu dem Tragrahmen (TR) drehbar gelagert ist und einen ersten Satz von Drehrahmen-Komponenten (D1, D2) aufweist,
- wobei der Tragrahmen (TR) einen Lüfter (V), einen Druckkanal (P) und eine erste Tragrahmen-Komponente (N1) aufweist, wobei der Druckkanal (P) eine erste Druckkanalwand (W1) aufweist, die den Druckkanal (P) in einer ersten Axialrichtung (Z) begrenzt, wobei die erste Axialrichtung (Z) zur der Drehachse (DA) im Wesentlichen parallel ist, wobei die erste Druckkanalwand (W1) einen ersten Satz von drehrahmenseitigen Öffnungen (PZ) aufweist,
- wobei der Druckkanal (P) eine zweite Druckkanalwand (W2) aufweist, die den Druckkanal (P) in wenigstens einer Radialrichtung (X, Y) begrenzt, wobei die wenigstens eine Radialrichtung (X, Y) zu der Drehachse (DA) im Wesentlichen senkrecht ist, wobei die zweite Druckkanalwand (W2) eine erste Öffnung (P1) aufweist,
- wobei ein Luftstrom (L) mittels des Lüfters (V) erzeugt (M1) wird,
- wobei der Luftstrom (L) mittels des Druckkanals (P) entlang wenigstens eines Strömungspfads (LP) geführt (M2) wird, wobei der wenigstens eine Strömungspfad (LP) um die Drehachse (DA) herum gekrümmt ist,
- wobei ein erster Drehrahmen-Luftstrom (LZ) mittels des ersten Satzes von drehrahmenseitigen Öffnungen (PZ) von dem Luftstrom (L) abgezweigt (M3) wird,
- wobei der erste Satz von Drehrahmen-Komponenten (D1, D2) mittels des ersten Drehrahmen-Luftstroms (LZ) gekühlt (M4) wird,
- wobei ein erster Tragrahmen-Luftstrom (L1) mittels der ersten Öffnung (P1) von dem Luftstrom (L) abgezweigt (M5) wird,
- wobei die erste Tragrahmen-Komponente (N1) mittels des ersten Tragrahmen-Luftstroms (L1) gekühlt (M6) wird.

10. Verfahren nach Anspruch 9,
- wobei die erste Tragrahmen-Komponente (N1) ein elektronisches Gerät ist.

11. Verfahren nach Anspruch 9 oder 10,
- wobei der Drehrahmen (DR) eine erste Drehrahmenwand (WD) aufweist, die in der ersten Axialrichtung (Z) auf die erste Druckkanalwand (W1) folgend angeordnet ist,
- wobei der erste Drehrahmen-Luftstrom (LZ) mittels eines ersten Satzes von druckkanalseitigen Öffnungen (PD) der ersten Drehrahmenwand (WD) aufgenommen wird.

12. Verfahren nach einem der Ansprüche 9 bis 11,
- wobei der Drehrahmen (DR) einen zweiten Satz von Drehrahmen-Komponenten (D21, D22) aufweist,
- wobei der Druckkanal (P) eine dritte Druckkanalwand (W3) aufweist, die den Druckkanal (P) in einer zweiten Axialrichtung (Z2) begrenzt, wobei die zweite Axialrichtung (Z2) zu der Drehachse (DA) im Wesentlichen parallel ist und zu der ersten Axialrichtung (Z) entgegengesetzt gerichtet ist, wobei die dritte Druckkanalwand (W3) einen zweiten Satz von drehrahmenseitigen Öffnungen (PZ2) aufweist,
- wobei ein zweiter Drehrahmen-Luftstrom (LZ2) mittels des zweiten Satzes von drehrahmenseitigen Öffnungen (PZ2) von dem Luftstrom (L) abgezweigt wird,
- wobei der Drehrahmen (DR) eine zweite Drehrahmenwand (WD2) aufweist, die in der zweiten Axialrichtung (Z2) auf die dritte Druckkanalwand (W3) folgend angeordnet ist, wobei die zweite Drehrahmenwand (WD2) einen zweiten Satz druckkanalseitiger Öffnungen (PD2) aufweist,
- wobei der zweite Drehrahmen-Luftstrom (LZ2) mittels des zweiten Satzes druckkanalseitiger Öffnungen (PD2) aufgenommen wird,
- wobei der zweite Satz von Drehrahmen-Komponenten (D21, D22) mittels des zweiten Drehrahmen-Luftstroms (LZ2) gekühlt wird.

13. Verfahren nach einem der Ansprüche 9 bis 12,
- wobei der Tragrahmen (TR) eine zweite Tragrahmen-Komponente (N2) aufweist, wobei die zweite Druckkanalwand (W2) eine zweite Öffnung (P2) aufweist,
- wobei ein zweiter Tragrahmen-Luftstrom (L2) mittels der zweiten Öffnung (P2) von dem Luftstrom (L) abgezweigt wird, wobei die erste Öffnung (P1) und die zweite Öffnung (P2) entlang des wenigstens einen Strömungspfades (LP) aufeinanderfolgend angeordnet sind,
- wobei die zweite Tragrahmen-Komponente (N2) mittels des zweiten Tragrahmen-Luftstroms (L2) gekühlt wird.

14. Verfahren nach einem der Ansprüche 9 bis 13,
- wobei der Tragrahmen (TR) einen Kipprahmen (KR), ein Kipplager (KL) und einen Stützrahmen (SR) aufweist, wobei der Kipprahmen (KR) mittels des Kipplagers (KL) um eine Kippachse (KA) relativ zu dem Stützrahmen (SR) kippbar gelagert ist,
- wobei der Kipprahmen (KR) den Lüfter (V) und den Druckkanal (P) aufweist, wobei der Drehrahmen (DR) mittels des Drehlagers (DL) um eine Drehachse (DA) relativ zu dem Kipprahmen (KR) drehbar gelagert ist,
- wobei der Stützrahmen (SR) die erste Tragrahmen-Komponente (N1) aufweist,
- wobei die erste Tragrahmen-Komponente (N1) mit der ersten Öffnung (P1) mittels einer Leitung (PN) zum Übertragen des ersten Tragrahmen-Luftstroms (L1) verbunden ist, wobei die Leitung (PN) einen flexiblen Abschnitt und/oder eine Drehdurchführung aufweist,
- wobei eine Kippbewegung des Kipprahmens (KR) relativ zu dem Stützrahmen (SR) ausgeführt wird,
- wobei die Leitung (PN) mittels des flexiblen Abschnitts und/oder der Drehdurchführung der Kippbewegung des Kipprahmens (KR) relativ zu dem Stützrahmen (SR) folgt.

15. Verfahren nach einem der Ansprüche 9 bis 14,
- wobei die Gantry (20) eine Verkleidung (C) zur Abgrenzung eines Innenbereichs (4) der Gantry (20) gegen eine Umgebung (5) der Gantry aufweist,
- wobei der Innenbereich (4) der Gantry (20) einen Zwischenraum (40) aufweist, wobei sich der Zwischenraum (40) zwischen dem Tragrahmen (TR) und dem Drehrahmen (DR) einerseits und der Verkleidung (C) andererseits erstreckt,
- wobei mittels des Zwischenraums (40) ein von dem ersten Satz von Drehrahmen-Komponenten (D1, D2) kommender Warmluftstrom und ein von der ersten Tragrahmen-Komponente (N1) kommender Warmluftstrom aufgenommen und zu dem Lüfter (V) geführt werden.

## Claims

1. Gantry (20) for a computed tomography device (1),
- wherein the gantry (20) has a carrier frame (TR), a pivot bearing (DL) and a rotating frame (DR),
- wherein the rotating frame (DR) is mounted by means of the pivot bearing (DL) such that it can rotate about an axis of rotation (DA) relative to the carrier frame (TR) and has a first set of rotating frame components (D1, D2),
- wherein the carrier frame (TR) has a fan (V) for generating an air flow (L), a pressure channel (P) for guiding the air flow (L) along at least one flow path (LP) and a first carrier frame component (N1), wherein the at least one flow path (LP) is curved around the axis of rotation (DA),
- wherein the pressure channel (P) has a first pressure channel wall (W1), which delimits the pressure channel (P) in a first axial direction (Z), wherein the first axial direction (Z) is substantially in parallel with the axis of rotation (DA), wherein the first pressure channel wall (W1) has a first set of rotating frame-side openings (PZ), which are configured to branch off a first rotating frame air flow (LZ) from the air flow (L), wherein the first rotating frame air flow (LZ) is provided for cooling the first set of rotating frame components (D1, D2),
- wherein the pressure channel (P) has a second pressure channel wall (W2), which delimits the pressure channel (P) in at least one radial direction (X, Y), wherein the at least one radial direction (X, Y) is substantially perpendicular to the axis of rotation (DA), **characterised in that**
the second pressure channel wall (W2) has a first opening (P1), which is configured to branch off a first carrier frame air flow (L1) from the air flow (L), wherein the first carrier frame air flow (L1) is provided for cooling the first carrier frame component (N1).

2. Gantry (20) according to claim 1,
- wherein the first set of rotating frame-side openings (PZ) contains at least one rotating frame-side opening, which is arranged along the at least one flow path (LP) downstream of the first opening (P1),
- wherein the first set of rotating frame-side openings (PZ) contains at least one rotating frame-side opening, which is arranged along the at least one flow path (LP) upstream of the first opening (P1).

3. Gantry (20) according to claim 1 or 2,
- wherein the first carrier frame component (N1) is an electronic device.

4. Gantry (20) according to one of claims 1 to 3,
- wherein the rotating frame (DR) has a first rotating frame wall (WD), which is arranged such that it follows the first pressure channel wall (W1) in the first axial direction (Z),
- wherein the first rotating frame wall (WD) has a first set of pressure channel-side openings (PD), which are configured to receive the first rotating frame air flow (LZ).

5. Gantry (20) according to one of claims 1 to 4,
- wherein the rotating frame (DR) has a second set of rotating frame components (D21, D22),
- wherein the pressure channel (P) has a third pressure channel wall (W3), which delimits the pressure channel (P) in a second axial direction (Z2), wherein the second axial direction (Z2) is substantially in parallel with the axis of rotation (DA) and is in the opposite direction to the first axial direction (Z), wherein the third pressure channel wall (W3) has a second set of rotating frame-side openings (PZ2), which are configured to branch off a second rotating frame air flow (LZ2) from the air flow (L), wherein the second rotating frame air flow (LZ2) is provided for cooling the second set of rotating frame components (D21, D22),
- wherein the rotating frame (DR) has a second rotating frame wall (WD2), which is arranged such that it follows the third pressure channel wall (W3) in the second axial direction (Z2),
- wherein the second rotating frame wall (WD2) has a second set of pressure channel-side openings (PD2), which are configured to receive the second rotating frame air flow (LZ2) .

6. Gantry (20) according to one of claims 1 to 5,
- wherein the carrier frame (TR) has a second carrier frame component (N2),
- wherein the second pressure channel wall (W2) has a second opening (P2), which is configured to branch off a second carrier frame air flow (L2) from the air flow (L), wherein the second carrier frame air flow (L2) is provided for cooling the second carrier frame component (N2),
- wherein the first opening (P1) and the second opening (P2) are arranged such that they follow one another along the at least one flow path (LP).

7. Gantry (20) according to claim 6,
- wherein the first set of rotating frame-side openings (PZ) contains at least one rotating frame-side opening, which is arranged along the at least one flow path (LP) between the first opening (P1) and the second opening (P2).

8. Gantry (20) according to one of claims 1 to 7,
- wherein the carrier frame (TR) has a tilting frame (KR), a tilting bearing (KL) and a supporting frame (SR), wherein the tilting frame (KR) is mounted by means of the tilting bearing (KL) such that it can tilt about a tilt axis (KA) relative to the supporting frame (SR),
- wherein the tilting frame has the fan (V) and the pressure channel (P), wherein the rotating frame (DR) is mounted by means of the pivot bearing (DL) such that it can rotate about an axis of rotation (DA) relative to the tilting frame (KR),
- wherein the supporting frame (SR) has the first carrier frame component (N1),
- wherein the first carrier frame component (N1) is connected to the first opening (P1) by means of a line (PN) for transferring the first carrier frame air flow (L1), wherein the line (PN) has a flexible section and/or a rotary feed-through, in order to be able to follow a tilting movement of the tilting frame (KR) relative to the supporting frame (SR).

9. Method for cooling a computed tomography device (1),
- wherein the computed tomography device (1) has a gantry (20) with a carrier frame (TR), a pivot bearing (DL) and a rotating frame (DR),
- wherein the rotating frame (DR) is mounted by means of the pivot bearing (DL) such that it can rotate about an axis of rotation (DA) relative to the carrier frame (TR) and has a first set of rotating frame components (D1, D2),
- wherein the carrier frame (TR) has a fan (V), a pressure channel (P) and a first carrier frame component (N1), wherein the pressure channel (P) has a first pressure channel wall (W1), which delimits the pressure channel (P) in a first axial direction (Z), wherein the first axial direction (Z) is substantially in parallel with the axis of rotation (DA), wherein the first pressure channel wall (W1) has a first set of rotating frame-side openings (PZ),
- wherein the pressure channel (P) has a second pressure channel wall (W2), which delimits the pressure channel (P) in at least one radial direction (X, Y), wherein the at least one radial direction (X, Y) is substantially perpendicular to the axis of rotation (DA), wherein the second pressure channel wall (W2) has a first opening (P1),
- wherein an air flow (L) is generated (M1) by means of the fan (V),
- wherein the air flow (L) is guided (M2) along at least one flow path (LP) by means of the pressure channel (P), wherein the at least one flow path (LP) is curved around the axis of rotation (DA),
- wherein a first rotating frame air flow (LZ) is branched off (M3) from the air flow (L) by means of the first set of rotating frame-side openings (PZ),
- wherein the first set of rotating frame components (D1, D2) are cooled (M4) by means of the first rotating frame air flow (LZ),
- wherein a first carrier frame air flow (L1) is branched off (M5) from the air flow (L) by means of the first opening (P1),
- wherein the first carrier frame component (N1) is cooled (M6) by means of the first carrier frame air flow (L1).

10. Method according to claim 9,
- wherein the first carrier frame component (N1) is an electronic device.

11. Method according to claim 9 or 10,
- wherein the rotating frame (DR) has a first rotating frame wall (WD), which is arranged such that it follows the first pressure channel wall (W1) in the first axial direction (Z),
- wherein the first rotating frame air flow (LZ) is received by means of a first set of pressure channel-side openings (PD) of the first rotating frame wall (WD),

12. Method according to one of claims 9 to 11,
- wherein the rotating frame (DR) has a second set of rotating frame components (D21, D22),
- wherein the pressure channel (P) has a third pressure channel wall (W3), which delimits the pressure channel (P) in a second axial direction (Z2), wherein the second axial direction (Z2) is substantially in parallel with the axis of rotation (DA) and is in the opposite direction to the first axial direction (Z), wherein the third pressure channel wall (W3) has a second set of rotating frame-side openings (PZ2),
- wherein a second rotating frame air flow (LZ2) is branched off from the air flow (L) by means of the second set of rotating frame-side openings (PZ2),
- wherein the rotating frame (DR) has a second rotating frame wall (WD2), which is arranged such that it follows the third pressure channel wall (W3) in the second axial direction (Z2), wherein the second rotating frame wall (WD2) has a second set of pressure channel-side openings (PD2),
- wherein the second rotating frame air flow (LZ2) is received by means of the second set of pressure channel-side openings (PD2),
- wherein the second set of rotating frame components (D21, D22) is cooled by means of the second rotating frame air flow (LZ2) .

13. Method according to one of claims 9 to 12,
- wherein the carrier frame (TR) has a second carrier frame component (N2), wherein the second pressure channel wall (W2) has a second opening (P2),
- wherein a second carrier frame air flow (L2) is branched off from the air flow (L) by means of the second opening (P2), wherein the first opening (P1) and the second opening (P2) are arranged such that they follow one another along the at least one flow path (LP),
- wherein the second carrier frame component (N2) is cooled by means of the second carrier frame air flow (L2).

14. Method according to one of claims 9 to 13,
- wherein the carrier frame (TR) has a tilting frame (KR), a tilting bearing (KL) and a supporting frame (SR), wherein the tilting frame (KR) is mounted by means of the tilting bearing (KL) such that it can tilt about a tilt axis (KA) relative to the supporting frame (SR),
- wherein the tilting frame (KR) has the fan (V) and the pressure channel (P), wherein the rotating frame (DR) is mounted by means of the pivot bearing (DL) such that it can rotate about an axis of rotation (DA) relative to the tilting frame (KR),
- wherein the supporting frame (SR) has the first carrier frame component (N1),
- wherein the first carrier frame component (N1) is connected to the first opening (P1) by means of a line (PN) for transferring the first carrier frame air flow (L1), wherein the line (PN) has a flexible section and/or a rotary feed-through,
- wherein a tilting movement of the tilting frame (KR) relative to the supporting frame (SR) is carried out,
- wherein the line (PN) follows the tilting movement of the tilting frame (KR) relative to the supporting frame (SR) by means of the flexible section and/or the rotary feed-through.

15. Method according to one of claims 9 to 14,
- wherein the gantry (20) has a cladding (C) for delimiting an inner region (4) of the gantry (20) in relation to a surrounding area (5) of the gantry,
- wherein the inner region (4) of the gantry (20) has an intermediate space (40), wherein the intermediate space (40) extends between the carrier frame (TR) and the rotating frame (DR) on one side and the cladding (C) on the other side,
- wherein a warm air flow coming from the first set of rotating frame components (D1, D2) and a warm air flow coming from the first carrier frame component (N1) are received by means of the intermediate space (40) and guided to the fan (V) .

## Revendications

1. Portique (20) d'un appareil (1) de tomodensitométrie assisté par ordinateur,
- dans lequel le portique (20) a un cadre (TR) porteur, un coussinet (DL) de pivotement et un cadre (DR) tournant,
- dans lequel le cadre (DR) tournant est, au moyen du coussinet (DL) de pivotement, monté tournant par rapport au cadre (TR) porteur autour d'un axe (DA) de rotation et a un premier ensemble de composants (D1, D2) de cadre tournant,
- dans lequel le cadre (TR) porteur a un ventilateur (V) de production d'un courant (L) d'air, un conduit (P) sous pression de conduite du courant (L) d'air le long d'au moins un chemin (LP) d'écoulement et un premier composant (N1) de cadre porteur, dans lequel le au moins un chemin (LP) d'écoulement est incurvé autour de l'axe (DA) de rotation,
- dans lequel le conduit (P) sous pression a une première paroi (W1) de conduit sous pression, qui délimite le conduit (P) sous pression dans une première direction (Z) axiale, dans lequel la première direction (Z) axiale est sensiblement parallèle à l'axe (DA) de rotation, dans lequel la première paroi (W1) du conduit sous pression a un premier ensemble d'ouvertures (PZ) du côté du cadre tournant, qui est agencé pour dévier, du courant (L) d'air, un premier courant (LZ) d'air de cadre de rotation, dans lequel le premier courant (LZ) d'air de cadre de rotation est prévu pour le refroidissement du premier ensemble de composants (D1, D2) de cadre de rotation,
- dans lequel le conduit (P) sous pression a une deuxième paroi (W2) de conduit sous pression, qui délimite le conduit (P) sous pression dans au moins une direction (X, Y) radiale, dans lequel la au moins une direction (X, Y) radiale est sensiblement perpendiculaire à l'axe (DA) de rotation, **caractérisé en ce que** la deuxième paroi (W2) de conduit sous pression a une première ouverture (P1), qui est agencée pour dévier du courant (L) d'air, un premier courant (L1) d'air de cadre porteur, dans lequel le premier courant (L1) d'air de cadre porteur est prévu pour le refroidissement du premier composant (N1) de cadre porteur.

2. Portique (20) suivant la revendication 1,
- dans lequel le premier ensemble d'ouvertures (PZ) du côté du cadre tournant comporte au moins une ouverture du côté du cadre tournant, qui est disposée, le long du au moins un chemin (LP) d'écoulement, après la première ouverture (P1),
- dans lequel le premier ensemble d'ouvertures (PZ) du côté du cadre tournant comporte au moins une ouverture du côté du cadre tournant, qui est disposée, le long du au moins un chemin (LP) d'écoulement, avant la première ouverture (P1).

3. Portique (20) suivant la revendication 1 ou 2,
- dans lequel le premier composant (N1) de cadre porteur est un appareil électronique.

4. Portique (20) suivant l'une des revendications 1 à 3,
- dans lequel le cadre (DR) tournant a une première paroi (WD) de cadre tournant, qui est disposée dans la première direction (Z) axiale en suivant la première paroi (W1) du conduit sous pression,
- dans lequel la première paroi (WD) de cadre tournant a un premier ensemble d'ouvertures (PD) du côté du conduit sous pression, qui est agencé pour recevoir le premier courant (LZ) d'air de cadre tournant.

5. Portique (20) suivant l'une des revendications 1 à 4,
- dans lequel le cadre (DR) tournant a un deuxième ensemble de composants (D21, D22) de cadre tournant,
- dans lequel le conduit (P) sous pression a une troisième paroi (W3) de conduit sous pression, qui délimite le conduit (P) sous pression dans une deuxième direction (Z2) axiale, dans lequel la deuxième direction (Z2) axiale est sensiblement parallèle à l'axe (DA) de rotation et est dirigée en sens contraire à la première direction (Z) axiale, dans lequel la troisième paroi (W3) du conduit sous pression a un deuxième ensemble d'ouvertures (PZ2) du côté du cadre tournant, qui dérive, du courant (L) d'air, un deuxième courant (LZ2) d'air de cadre tournant, dans lequel le deuxième courant (LZ2) d'air de cadre tournant est prévu pour le refroidissement du deuxième ensemble de composants (D21, D22) de cadre tournant,
- dans lequel le cadre (DR) tournant a une deuxième paroi (WD2) de cadre tournant, qui est disposée, dans la deuxième direction (Z2) axiale, en suivant la troisième paroi (W3) du conduit sous pression,
- dans lequel la deuxième paroi (WD2) du cadre tournant a un deuxième ensemble d'ouvertures (PD2) du côté du conduit sous pression, qui est agencé pour recevoir le deuxième courant (LZ2) d'air de cadre tournant.

6. Portique (20) suivant l'une des revendications 1 à 5,
- dans lequel le cadre (TR) porteur a un deuxième composant (N2) de cadre porteur,
- dans lequel la deuxième paroi (W2) du conduit sous pression a une deuxième ouverture (P2), qui est agencée pour dévier, du courant (L) d'air, un deuxième courant (L2) d'air de cadre porteur, dans lequel le deuxième courant (L2) d'air de cadre porteur est prévu pour le refroidissement du deuxième composant (N2) de cadre porteur,
- dans lequel la première ouverture (P1) et la deuxième ouverture (P2) sont disposées en se suivant l'une l'autre le long du au moins un chemin (LP) d'écoulement.

7. Portique (20) suivant la revendication 6,
- dans lequel le premier ensemble d'ouvertures (PZ) du côté du cadre tournant comporte au moins une ouverture du cadre tournant, qui est disposée, le long du au moins un chemin (LP) d'écoulement, entre la première ouverture (P1) et la deuxième ouverture (P2).

8. Portique (20) suivant l'une des revendications 1 à 7,
- dans lequel le cadre (TR) porteur comporte un cadre (KR) basculant, un palier (KL) de basculement et un cadre (SR) d'appui, dans lequel le cadre (KR) basculant est monté au moyen du palier (KL) de basculement basculant autour d'un axe (KA) de basculement par rapport au cadre (SR) d'appui,
- dans lequel le cadre basculant a le ventilateur (V) et le conduit (P) sous pression, dans lequel le cadre (DR) tournant est, au moyen du coussinet (DL) de pivotement, monté tournant autour d'un axe (DA) par rapport au cadre (KR) basculant,
- dans lequel le cadre (SR) d'appui a le premier composant (N1) de cadre porteur,
- dans lequel le premier composant (N1) de cadre porteur est relié à la première ouverture (P1) au moyen d'un conduit (PN) pour la transmission du premier courant (L1) d'air de cadre porteur, dans lequel le conduit (PN) a un tronçon souple et/ou un passage tournant, afin de pouvoir suivre un mouvement de basculement du cadre (KR) basculant par rapport au cadre (SR) d'appui.

9. Procédé de refroidissement d'un appareil (1) de tomodensitométrie assisté par ordinateur,
- dans lequel l'appareil (1) de tomodensitométrie assisté par ordinateur a un portique (20) ayant un cadre (TR) porteur, un coussinet (DL) de pivotement et un cadre (DR) tournant,
- dans lequel le cadre (DR) tournant est, au moyen du coussinet (DL) de pivotement, monté tournant par rapport au cadre (TR) porteur autour d'un axe (DA) de rotation et a un premier ensemble de composants (D1, D2) de cadre tournant,
- dans lequel le cadre (TR) porteur a un ventilateur (V), un conduit (P) sous pression et un premier composant (N1) de cadre porteur, dans lequel le conduit (P) sous pression a une première paroi (W1) de conduit sous pression, qui délimite le conduit (P) sous pression dans une première direction (Z) axiale, dans lequel le première direction (Z) axiale est sensiblement parallèle à l'axe (DA) de rotation, dans lequel le première paroi (W1) de conduit sous pression a un premier ensemble d'ouvertures (PZ) du côté du cadre tournant,
- dans lequel le conduit (P) sous pression a une deuxième paroi (W2) de conduit sous pression, qui délimite le conduit (P) sous pression dans au moins une direction (X, Y) radiale, dans lequel la au moins une direction (X, Y) radiale est sensiblement perpendiculaire à l'axe (DA) de rotation, dans lequel la deuxième paroi (W2) du conduit sous pression a une première ouverture (P1),
- dans lequel on produit un courant (L) d'air au moyen du ventilateur (V),
- dans lequel on conduit (M2) le courant (L) d'air au moyen du conduit (P) sous pression le long d'au moins un chemin (LP) d'écoulement, dans lequel le au moins un chemin (LP) d'écoulement est incurvé autour de l'axe (DA) de rotation,
- dans lequel on dévie (M3), du courant (L) d'air, un premier courant (LZ) d'air de cadre tournant au moyen du premier ensemble d'ouvertures (PZ) du côté du cadre de rotation,
- dans lequel on refroidit (M4) le premier ensemble de composants (D1, D2) de cadre de rotation au moyen du premier courant (LZ) d'air de cadre de rotation,
- dans lequel on dévie (M5), du courant (L) d'air, un premier courant (L1) d'air de cadre porteur au moyen de la première ouverture (P1),
- dans lequel on refroidit (M6) le premier composant (N1) de cadre porteur au moyen du premier courant (L1) d'air de cadre porteur.

10. Procédé suivant la revendication 9,
- dans lequel le premier composant (N1) de cadre porteur est un appareil électronique.

11. Procédé suivant la revendication 9 ou 10,
- dans lequel le cadre (DR) tournant a une première paroi (WD) de cadre tournant, qui est disposée dans la première direction (Z) axiale en suivant la première paroi (W1) du conduit sous pression,
- dans lequel on reçoit le premier courant (LZ) d'air de cadre tournant au moyen d'un premier ensemble d'ouvertures (PD), du côté du conduit sous pression, de la première paroi (WD) de cadre de rotation.

12. Procédé suivant l'une des revendications 9 à 11,
- dans lequel le cadre (DR) tournant a un deuxième ensemble de composants (D21, D22) de cadre tournant,
- dans lequel le conduit (P) sous pression a une troisième paroi (W3) de conduit sous pression, qui délimite le conduit (P) sous pression dans une deuxième direction (Z2) axiale, dans lequel la deuxième direction (Z2) axiale est sensiblement parallèle à l'axe (DA) de rotation et est dirigée en sens contraire à la première direction (Z) axiale, dans lequel la troisième paroi (W3) du conduit sous pression a un deuxième ensemble d'ouvertures (PZ2) du côté du cadre tournant,
- dans lequel on dévie, du courant (L) d'air, un deuxième courant (LZ2) d'air du cadre tournant, au moyen d'un deuxième ensemble d'ouvertures (PZ2) du côté du cadre tournant,
- dans lequel le cadre (DR) tournant a une deuxième paroi (WD2) de cadre tournant, qui est disposée, dans la deuxième direction (Z2) axiale, en suivant la troisième paroi (W3) du canal sous pression, dans lequel la deuxième paroi (WD2) de cadre tournant a un deuxième ensemble d'ouvertures (PD2) du côté du conduit sous pression,
- dans lequel on reçoit le deuxième courant (LZ2) d'air de cadre tournant au moyen du deuxième ensemble d'ouvertures (PD2) du côté du conduit sous pression,
- dans lequel on refroidit le deuxième ensemble de composants (D21, D22) de cadre tournant au moyen du deuxième courant (LZ2) d'air de cadre tournant.

13. Procédé suivant l'une des revendications 9 à 12,
- dans lequel le cadre (TR) porteur a un deuxième composant (N2) de cadre porteur, dans lequel la deuxième paroi (W2) du canal sous pression a une deuxième ouverture (P2),
- dans lequel on dévie, du courant (L) d'air, un deuxième courant (L2) d'air de cadre porteur au moyen de la deuxième ouverture (P2), dans lequel la première ouverture (P1) et la deuxième ouverture (P2) sont disposées à la suite l'une de l'autre le long du au moins un chemin (LP) d'écoulement,
- dans lequel on refroidit le deuxième composant (N2) de cadre porteur au moyen du deuxième courant (L2) d'air de cadre porteur.

14. Procédé suivant l'une des revendications 9 à 13,
- dans lequel le cadre (TR) porteur comporte un cadre (KR) basculant, un palier (KL) de basculement et un cadre (SR) d'appui, dans lequel le cadre (KR) basculant est monté au moyen du palier (KL) de basculement basculant autour d'un axe (KA) de basculement par rapport au cadre (SR) d'appui,
- dans lequel le cadre (KR) basculant a le ventilateur (V) et le conduit (P) sous pression, dans lequel le cadre (DR) tournant est, au moyen du coussinet (DL) de pivotement, monté tournant autour d'un axe (DA) par rapport au cadre (KR) basculant,
- dans lequel le cadre (SR) d'appui a le premier composant (N1) de cadre porteur,
- dans lequel le premier composant (N1) de cadre porteur est relié à la première ouverture (P1) au moyen d'un conduit (PN) pour la transmission du premier courant (L1) d'air de cadre porteur, dans lequel le conduit (PN) a un tronçon souple et/ou un passage tournant,
- dans lequel on effectue un mouvement de basculement du cadre (KR) basculant par rapport au cadre (SR) d'appui,
- dans lequel le conduit (PN) suit, au moyen du tronçon souple et/ou du passage tournant, le mouvement du cadre (KR) basculant par rapport au cadre (SR) d'appui.

15. Procédé suivant l'une des revendications 9 à 14,
- dans lequel le portique (20) a un habillage (C) de délimitation d'une partie (4) intérieure du portique (20) par rapport à ce qui entoure (5) le portique,
- dans lequel la partie (4) intérieure du portique (20) a un espace (40) intermédiaire, dans lequel l'espace (40) intermédiaire s'étend entre le cadre (TR) porteur et le cadre (DR) tournant d'une part et l'habillage (C) d'autre part,
- dans lequel, au moyen de l'espace (40) intermédiaire, on reçoit un courant d'air chaud venant du premier ensemble de composants (D1, D2) de cadre tournant et un courant d'air chaud venant du premier composant (N1) de cadre porteur et on les envoie au ventilateur (V).
